# FASCICULE DE BREVET EUROPEEN

(11) **EP 0 605 321 B1**
(45) Date de publication et mention de la délivrance du brevet: **08.10.1997**
(21) Numéro de dépôt: 93403191.5
(22) Date de dépôt: 28.12.1993
(51) Int. Cl.: A61K 7/22, A61K 7/16

(54) **Composition antibactérienne à usage buccal destinée à éviter la formation de la plaque dentaire**
Antibakterielles Mundpflegemittel gegen Zahnbelagbildung
Antibacterial buccal composition to avoid the formation of dental plaque

(30) Priorité: 30.12.1992 FR 9215946
(43) Date de publication de la demande: 06.07.1994
(73) Titulaire: L'OREAL, 75008 Paris (FR)
(72) Inventeur: Darmenton, Patrick, F-92320 Bourg-la-Reine (FR); Sterle, Pascal, F-77330 Ozoir-la-Ferrière (FR)
(74) Mandataire: Casalonga, Axel

(56) Documents cités:
- WO-A-92/20319
- GB-A- 2 140 691

## Description

La présente invention concerne des compositions à usage buccal destinées à éviter la formation de la plaque dentaire.

Dans le domaine de l'hygiène buccale, de la prévention de la carie dentaire, de la prévention et du traitement des maladies parodontales comme la gingivite et la parodontite, il est connu que le contrôle de la plaque bactérienne et la lutte contre cette plaque sont des éléments essentiels.

Parmi les agents antibactériens usuellement utilisés pour prévenir la formation de la plaque bactérienne dentaire, la chlorhexidine est connue pour être l'un des antiseptiques les plus efficaces vis-à-vis de la flore buccale. L'intérêt de la chlorhexidine a été reconnu au stade initial de la gingivite ainsi qu'au stade plus tardif des parodontolyses. Elle inhibe, en effet, les protéases d'origine bactérienne et, dans les inflammations gingivales, modulerait la formation des radicaux libres par les polynucléaires,

Cependant, l'utilisation de la chlorhexidine présente certains inconvénients. L'un de ses effets secondaires est la coloration des dents. Le goût amer que présente les produits formulés avec la chlorhexidine en est un autre.

Afin d'éviter ces deux inconvénients, on a proposé d'employer la chlorhexidine sous forme d'un complexe chlorhexidine-polymère anionique (US-4.980.150).

En l'absence d'autres agents aussi actifs contre la plaque, il est apparu un besoin de formuler la chlorhexidine à des concentrations minorées pour une efficacité équivalente, voire supérieure, afin d'éliminer les effets secondaires dus à la chlorhexidine.

La demanderesse a maintenant mis en évidence que certains mélanges de tensio-actifs, associés à la chlorhexidine, permettent de potentialiser son activité anti-plaque et qu'il était ainsi possible de formuler la chlorhexidine à des doses moindres, tout en conservant une bonne activité antibactérienne.

Ainsi, la présente invention concerne des compositions à usage buccal comportant de la chlorhexidine et/ou l'un au moins de ses sels, associée à un tensio-actif anionique et à un tensio-actif non-ionique. Ces compositions sont caractérisées en ce qu'elles comportent une quantité a inférieure à 1% en poids de dodécanediol polyglycérolé à 3,5 moles de glycérol et une quantité b inférieure à 2% en poids de laurylsulfate de sodium, le rapport a/b étant compris entre 0,1 et 1 non inclus et la somme a + b étant inférieure à 2% en poids, par rapport au poids total de la composition.

Le laurylsulfate de sodium est un tensio-actif connu pour son utilisation dans la formulation de produits d'hygiène bucco-dentaire et notamment de pâte dentifrice.

Par ailleurs, le dodécanediol polyglycérolé à 3,5 moles de glycérol est connu notamment du document de brevet français FR-2.091.516, et son utilisation dans la formulation de produits de soin de la bouche et des dents est connue par le document de brevet français FR-2.546.751.

Comme cela apparaîtra dans les exemples, l'association selon l'invention de la chlorhexidine et/ou de l'un au moins de ses sels avec le mélange des deux tensio-actifs que sont le laurylsulfate de sodium et le dodécanediol polyglycérolé permet, de façon surprenante, d'obtenir un effet antiplaque de la chlorhexidine potentialisé par son association avec les deux tensio-actifs, dans les conditions pondérales évoquées ci-dessus.

De préférence, selon l'invention, le rapport pondéral a/b est compris entre 0,2 et 0,5.

Par sels de la chlorhexidine, on entend les digluconate, diformiate, diacétate, dipropionate, dichlorhydrate, dilactate, dinitrate, sulfate et tartrate de chlorhexidine; de préférence on utilise le digluconate, le dichlorhydrate ou le diacétate de chlorhexidine.

On utilise plus particulièrement le digluconate de chlorhexidine vendu, par exemple, en solution aqueuse à 20% de matières actives par la Société ICI sous la dénomination commerciale "HIBITANE" ou "ARLACID G".

Selon l'invention, la composition antibactérienne comporte de 0,01 à 0,6% en poids de chlorhexidine MA (matière active), de préférence elle en contient 0,06 g pour 100 g de composition.

Les compositions de l'invention peuvent se présenter sous les diverses formes usuelles pour les compositions à usage buccal et notamment sous forme de pâtes ou gels dentifrices, de bains de bouche, de sprays, de mousses, de gargarismes, de gels dentaires ou de gommes à mâcher, le véhicule étant alors choisi selon la forme désirée.

Les compositions à usage buccal de l'invention peuvent contenir, outre l'association de la chlorhexidine et/ou de l'un de ses sels avec les tensio-actifs particuliers, à titre de véhicule ou pour leur activité propre, des excipients ou ingrédients habituellement utilisés dans les produits à usage buccal sous la forme correspondante.

Ces compositions sont préparées selon les procédés usuels correspondants aux véhicules choisis. Le véhicule physiologiquement acceptable peut être de différente nature selon la forme choisie pour la composition : solution aqueuse, solution hydroalcoolique épaissie ou non, gomme, excipient pâteux ou solide ...

Selon les formes désirées, elles peuvent contenir en particulier au moins un agent de polissage dans des proportions allant jusqu'à 95%. Les agents de polissages sont des abrasifs qui peuvent être d'origine minérale ou organique. Leur nature peut différer selon le véhicule utilisé pour la forme choisie, comme cela apparaîtra ci-dessous.

Dans certaines formes, les compositions peuvent éventuellement contenir, en plus des tensio-actifs particuliers faisant l'objet de l'invention, un ou plusieurs agent(s) tensio-actif(s) suffisamment stables et moussants. Les agents tensio-actifs utilisables peuvent être de nature anionique, amphotère, zwitterionique, cationique ou non-ionique.

De façon générale, les tensio-actifs dans leur ensemble peuvent être présents dans une gamme pondérale allant jusqu'à 20% par rapport à la composition et particulièrement dans la gamme de 0,5 à 5%.

En outre, les compositions selon l'invention peuvent contenir d'autres agents actifs pour l'hygiène buccale et notamment des agents connus pour détruire la mauvaise haleine, tels que par exemple les cyclodextrines ou des composés du zinc qui sont des sels minéraux ou organiques tels que par exemple les halogénures de zinc, l'acétate de zinc, le citrate de zinc ou le fluorure de zinc.

Par ailleurs, elles peuvent contenir d'autres agents, usuels dans les compositions buccales, tels que les agents de cohésion, les agents épaississants, les agents antibiotiques, les agents édulcorants, humectants ou rafraîchissants, les agents conservateurs, les agents sucrants, les colorants, les arômes, les substances et agents aromatisants ou de sapidité, les agents peptisants, les agents plastifiants, les agents antibactériens ou bactéricides différents de la chlorhexidine et de ses sels, des vitamines, les agents anticaries, les agents antitartre, les cicatrisants, les vasomoteurs, les agents antisaignement, les agents actifs sur la gencive et les agents de polissage. Ces différents agents sont présents dans la composition selon l'invention notamment selon la forme qu'elle prend.

Ainsi, lorsque la composition à usage buccal est un spray, le véhicule peut être une solution hydro-alcoolique et la composition peut contenir en outre des arômes, des agents peptisants, des agents édulcorants, humectants ou rafraîchissants.

De même, à titre d'exemple, lorsque la composition est sous forme de bains de bouche, le véhicule peut être essentiellement constitué par une solution aqueuse des deux tensio-actifs objets de l'invention, éventuellement avec d'autres agents tensio-actifs décrits ci-dessus, et/ou un agent épaississant, et peut comporter en outre des agents bactéricides différents de la chlorhexidine et de ses sels, des édulcorants et des substances aromatisantes.

En outre, lorsque les compositions sont sous forme de gargarismes, elles peuvent renfermer un agent actif du type antibiotique.

A titre d'exemple, lorsque la composition est sous forme de gel dentaire, elle peut en outre contenir des agents actifs sur la gencive.

Lorsque les compositions sont sous forme de gomme à mâcher, elles contiennent au moins une gomme masticable naturelle ou synthétique, et peuvent contenir des agents plastifiants, des vitamines, des agents d'aromatisation ou de sapidité, des agents sucrants, des agents humectants, des bactéricides différents de la chlorhexidine ou de ses sels, des conservateurs, des colorants et éventuellement des agents de polissage.

Parmi les gommes ayant une élasticité suffisante, seules ou en mélanges, pour être masticables, on peut citer parmi les gommes naturelles le latex d'Hévéa, la gomme chicle, la gomme schulong et parmi les gommes de synthèse, l'acétate de polyvinyle et les élastomères de synthèse caoutchouc silicone, caoutchouc butyl. Généralement, ces gommes contiennent 0,5 à 70% en poids de gomme masticable.

Des agents de polissage peuvent être éventuellement utilisés selon l'invention. Pour les gommes à mâcher, on peut employer alors des agents de polissage usuels pour les gommes à mâcher, d'origine minérale ou organique. Ce sont par exemple les carbonate de calcium, de magnésium, de sodium, les phosphates et sulfates de calcium, l'alumine et l'alumine hydratée, les silices, les oxydes, hydroxydes, trisilicates et pyrophosphates de magnésium, ou des composés cellulosiques obtenus par broyage de graines de céréales.

Lorsque les compositions sont sous forme de pâte dentifrice, elles peuvent contenir un agent de polissage dans des proportions pouvant aller de 2 à 70% environ, et de préférence 15 à 25%. Il s'agit en général d'un matériau de polissage abrasif minéral constitué par un ou plusieurs composés, en grande partie insolubles dans l'eau. On peut citer par exemple les métaphosphates de sodium ou de potassium, le phosphate de calcium dihydraté, le phosphate dicalcique, le phosphate tricalcique, le pyrophosphate de calcium, l'alumine, les alumines hydratées et en particulier trihydratées, les silices, les silicates d'aluminium ou de zirconium, la bentonite ainsi que l'orthophosphate de magnésium ou le phosphate trimagnésien.

Lorsque la composition est sous forme de gel transparent, à titre d'agents de polissage, on peut utiliser un agent de polissage à base de silice colloïdale ou d'aluminosilicates de métaux alcalins ou alcalino-terreux, et de préférence de sodium ou de calcium.

De plus, dans certaines formes, et notamment dans les pâtes dentifrices, les compositions selon l'invention peuvent contenir un ou plusieurs agents de cohésion. Ces agents de cohésion peuvent être incorporés dans des proportions pouvant aller jusqu'à 10% en poids par rapport au poids total de la composition, et de préférence de 0,5 à 3% en poids. Ceux-ci peuvent être choisis parmi les épaississants naturels tels que les alginates et les pectines, les gommes naturelles comme la gomme adragante, les gommes de xanthane, les gommes de guar, les gommes de caroube ou de carraghénanes ou des épaississants synthétiques, en général dérivés de cellulose comme le sel de sodium de la carboxyméthylcellulose, la méthylcellulose ou les hydroxy-alkylcelluloses ou des acides polyacryliques réticulés comme les "CARBOPOLS".

Dans les compositions à usage buccal de l'invention, on peut utiliser en outre un agent édulcorant. Parmi les agents édulcorants utilisables, on peut citer le saccharose, le lactose, le fructose, le xylitol, le cyclamate de sodium, le maltose ou le saccharinate de sodium, les glycyrrhizinates de sodium ou d'ammonium, les mélanges d'α-glucosyl/steviolglucoside, le D-Mannitol, l'Aspartame, l'Acesulfam K et leurs mélanges.

Ces agents édulcorants sont alors présents dans des concentrations allant jusqu'à 2%.

Comme agents humectants, on peut citer le sorbitol, le glycérol ou le xylitol, présents à ce titre dans des concentrations pouvant atteindre 70%.

En outre, les agents rafraîchissants, comme le menthol ou l'éthylmaltol peuvent être incorporés.

Dans les compositions selon l'invention, il est également possible d'utiliser des agents conservateurs pour assurer une bonne pureté bactériologique des formulations. Ceux-ci peuvent être choisis parmi le parahydroxybenzoate de méthyle, le parahydroxybenzoate de propyle, le benzoate de sodium, entre autres agents conservateurs usuels. Les concentrations peuvent alors aller jusqu'à 1% en poids.

Comme substance aromatisante pour les compositions selon l'invention, on peut citer les essences de menthe, d'anis, d'eucalyptus, de cannelle, de girofle, de sauge, de réglisse, ou de fruits comme le citron, l'orange, la mandarine ou la fraise; on peut éventuellement utiliser à ce titre le salicylate de méthyle. Les substances aromatisantes, quand elles sont utilisées, peuvent l'être jusqu'à 5% en poids de la composition.

Outre la chlorhexidine ou ses sels selon l'invention, on peut utiliser un agent antibactérien choisi de préférence parmi les huiles essentielles ou des substances telles que l'alexidine, l'octinidine, l'héxétidine, le phénoxyéthanol, l'alcool phénéthylique et le triclosan, dans des concentrations pondérales totales pouvant représenter jusqu'à 10% en poids de la composition.

Enfin, il est possible d'incorporer dans les compositions à usage buccal selon l'invention, des agents anticaries comme le monofluorophosphate de sodium, les fluorures de sodium ou d'étain, les amines fluorées, les fluorures de polymère cationique tels que ceux qui sont décrits dans le brevet français 2 647 012.

Enfin, les compositions peuvent contenir des agents actifs tels que des agents antitartre, des agents cicatrisants, des agents vasomoteurs ou des agents antisaignement, et des oligo-éléments tels que des sels de cuivre, de zinc et de magnésium.

### EXEMPLE I

### DETERMINATION DES CONCENTRATIONS MINIMALES INHIBITRICES (CMI)

On détermine les CMI du digluconate de chlorhexidine (CHX), du laurylsulfate de sodium (LSNa) et du dodécanediol polyglycérolé (DP), seul, puis celles du digluconate de chlorhexidine, en présence de différents mélanges LSNa + DP en milieu liquide.

### Conditions opératoires et méthode

| | | |
|---|---|---|
| - Milieu de culture utilisé | | Heart and Brain [Pasteur (Code 74 016) décrit dans CREITZ et PUCKETT, Am. J. Cli. Path., 1954 - 24 - 1318/1323] |
| - Souches | Staphylococcus aureus | C i P 53 154 |
| | Lactobacillus acidophilus | C i P 1627 |
| - Conditions d'incubation | | 37° ± 1°C pendant 48 heures |

La détermination se fait en milieu liquide en tube. La croissance bactérienne est évaluée de façon visuelle, en fonction du trouble du liquide dans les tubes, l'absence de trouble signifiant qu'il n'y a pas de croissance bactérienne. On prépare une série de tubes correspondant à une gamme de concentration en agent antibactérien, on ensemence par la souche bactérienne, on incube puis on évalue de façon visuelle les troubles obtenus. Le premier tube non trouble, en quantité croissante, permet de déterminer la CMI des produits testés (seuls ou en mélange) pour leurs propriétés antibactériennes.

### Résultats

**TABLEAU I**

| ESSAIS | CMI en % | |
|---|---|---|
| | Staphylococcus aureus | Lactobacillus acidophilus |
| Digluconate de chlorhexidine | < 0,0002 | 0,0002 |
| LSNa | < 0,01 | < 0,01 |
| DP | 0,05 | 0,05 |
| CHX + 1,5% DP | < 0,006 | < 0,006 |
| CHX + 1,5% LSNa | < 0,006 | < 0,006 |
| CHX + 1,25% DP + 0,25% LSNa | 0,04 | < 0,006 |
| CHX + 1,00% DP + 0,50% LSNa | 0,12 | < 0,006 |
| CHX + 0,75% DP + 0,75% LSNa | < 0,006 | < 0,006 |
| CHX + 0,50% DP + 1,00% LSNa | < 0,006 | < 0,006 |
| CHX + 0,25% DP + 1,25% LSNa | < 0,006 | < 0,006 |

*Tableau 1* : Détermination des concentrations minimales inhibitrices

### EXEMPLE II

### DETERMINATION DU POUVOIR BACTERICIDE DE LA CHLORHEXIDINE EN PRESENCE DES DIFFERENTS MELANGES DES DEUX TENSIO-ACTIFS

Le pouvoir bactéricide en présence de différents mélanges de LSNa et de DP, toujours à concurrence de 1,5% en concentration finale, a été étudié selon la méthode de diffusion décrite ci-après.

Ces essais ont été réalisés selon les directives fixées par l'arrêté du 3 juillet 1972 (Journal Officiel du 8 août 1972).

Cette étude a porté sur des souches particulièrement impliquées dans la formation de la plaque :

| | | |
|---|---|---|
| - Streptococcus mutans | ATCC | 25175 |
| - Actynomyces viscosus | CiP | 103147 |

### Préparation des suspensions microbiennes d'essai : culture sur milieu de Rosenow cystéiné régénéré et additionné de sang de cheval (2 ml). Incubation à 37°C (3 à 5 jours).

### Conditions opératoires

Après solidification sur une surface plane et horizontale et séchage sous hotte à flux laminaire d'air stérile, les géloses nutritives coulées en boîtes de pétri de 90 mm de diamètre sont ensemencées en surface par chacune des suspensions microbiennes d'essai essayées.

Des cupules de 8 mm de diamètre sont creusées à l'emporte pièce stérilisé par flambage.

Chaque cupule reçoit le produit ou une dilution de ce produit en eau distillée stérile.

Après incubation en anaérobiose à 37°C ± 1°C, les diamètres moyens des zones d'inhibition sont mesurées à ± 1 mm près.

### Interprétation

La présence d'un Pouvoir Inhibiteur est caractérisée par la présence d'une zone d'inhibition quantifiée de façon relative par son diamètre moyen.
- Milieux de culture utilisés pour les diffusions :
   Gélose Columbia [Pasteur (Code 64 677), décrit dans ELLNER & al., Am. J. Clin. Path., 1966 - 45 - 502/504], régénérée et additionnée de sang de cheval à 10% (v/v).
- Conditions d'incubation:
   37° ± 1°C pendant 3 à 5 jours en anaérobiose continue.

### Résultats

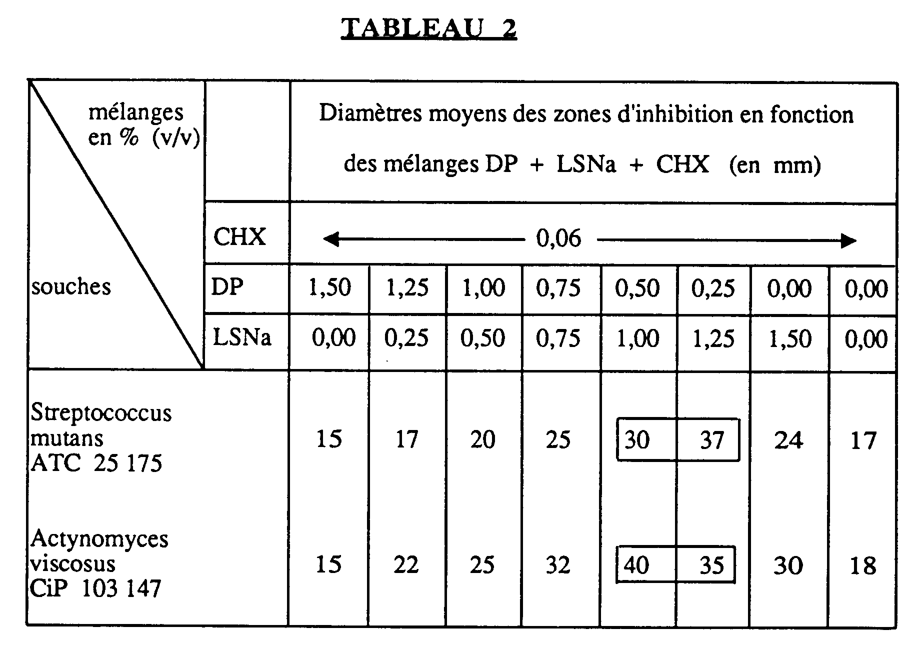

Il apparaît que les mélanges dont les résultats sont entourés ont un pouvoir bactéricide meilleur que la chlorhexidine seule, ou l'un ou l'autre des tensio-actifs, seul, en présence de chlorhexidine.

### EXEMPLES DE FORMULATION

### EXEMPLE 1

On prépare une pâte dentifrice antiplaque de composition suivante :

| | |
|---|---|
| - Sorbitol en solution aqueuse à 70% de MA | 21 g MA |
| - Carboxyméthylcellulose de sodium, vendue sous la dénomination "BLANOSE 9M 31 F" par la Société HERCULES | 0,8 g |
| - Carbonate de calcium | 50 g |
| - Saccharinate de sodium | 0,2 g |
| - Monofluorophosphate de sodium | 0,8 g |
| - Menthe poivrée | 1 g |
| - Digluconate de chlorhexidine en solution à 20% | 0,06 g MA |
| - Tensio-actif non-ionique de type hydroxypropyléther obtenu par condensation, en catalyse alcaline, de 3,5 moles de glycidol sur un mélange d'alphadiols en C₁₁-C₁₄ selon le brevet français n° 2.091.516 | 0,2 g |
| - Laurylsulfate de sodium | 1 g |
| - Benzoate de sodium | 0,1 g |
| - Eau purifiée qsp | 100 g |

### EXEMPLE 2

On prépare une pâte dentifrice antiplaque de composition suivante :

| | |
|---|---|
| - Silice précipitée abrasive, vendue sous la dénomination "TIXOSIL 53" par la Société RHONE POULENC | 15 g |
| - Silice précipitée gonflante, vendue sous la dénomination "TIXOSIL 333" par la Société RHONE POULENC | 8 g |
| - Sorbitol en solution aqueuse à 70% de MA | 19,6 g MA |
| - Carboxyméthylcellulose de sodium, vendue sous la dénomination "BLANOSE 9M 31 F" par la Société HERCULES | 1,2 g |
| - Saccharinate de sodium | 0,2 g |
| - Monofluorophosphate de sodium | 1,13 g |
| - Menthe | 0,8 g |
| - Dioxyde de titane | 0,8 g |
| - Digluconate de chlorhexidine en solution à 20% | 0,06 g MA |
| - Tensio-actif non-ionique de type hydroxy propyléther obtenu par condensation, en catalyse alcaline, de 3,5 moles de glycidol sur un mélange d'alphadiols en C₁₁-C₁₄ selon le brevet français n° 2.091.516 | 0,5 g |
| - Laurylsulfate de sodium | 1 g |
| - Parahydroxybenzoate de méthyle | 0,2 g |
| - Eau purifiée qsp | 100 g |

### EXEMPLE 3

On prépare une pâte dentifrice antiplaque de composition suivante :

| | |
|---|---|
| - Sorbitol en solution aqueuse à 70% de MA | 21 g MA |
| - Carboxyméthylcellulose de sodium, vendue sous la dénomination "BLANOSE 9M 31 F" par la Société HERCULES | 0,8 g |
| - Carbonate de calcium | 50 g |
| - Saccharinate de sodium | 0,2 g |
| - Monofluorophosphate de sodium | 0,8 g |
| - Menthe poivrée | 1 g |
| - Digluconate de chlorhexidine en solution à 20% | 0,06 g MA |
| - Tensio-actif non-ionique de type hydroxypropyléther obtenu par condensation, en catalyse alcaline, de 3,5 moles de glycidol sur un mélange d'alphadiols en C₁₁-C₁₄ selon le brevet français n° 2.091.516 | 0,25 g |
| - Laurylsulfate de sodium | 1,25 g |
| - Benzoate de sodium | 0,1 g |
| - Eau purifiée qsp | 100 g |

## Revendications

1. Composition à usage buccal comportant de la chlorhexidine dans un véhicule physiologiquement acceptable, caractérisée en ce que la chlorhexidine et/ou l'un au moins de ses sels est associée à la combinaison de :
- une quantité a inférieure à 1% en poids de dodécanediol polyglycérolé à 3,5 moles de glycérol, et
- une quantité b inférieure à 2% en poids de laurylsulfate de sodium,
le rapport a/b étant supérieur à 0,1 et inférieur à 1 et la somme a + b étant inférieure à 2% en poids par rapport au poids total de la composition.

2. Composition selon la revendication 1, caractérisée en ce que la composition comporte de 0,01 à 0,6 en poids de chlorhexidine et/ou de l'un au moins de ses sels en matière active par rapport au poids total de la composition.

3. Composition selon la revendication 1 ou 2, caractérisée en ce que les sels de chlorhexidine sont choisis parmi le digluconate, le diformiate, le diacétate, le dipropionate, le dichlorhydrate, le dilactate, le dinitrate, le sulfate et le tartrate de chlorhexidine.

4. Composition selon l'une des revendications 1 à 3, caractérisée en ce que les sels de chlorhexidine sont choisis parmi le digluconate, le dichlorhydrate et le diacétate de chlorhexidine.

5. Composition selon l'une des revendications 1 à 4, caractérisée en ce que le rapport a/b est compris entre 0,2 et 0,5.

6. Composition selon l'une des revendications 1 à 5, caractérisée en ce qu'elle est sous forme de gels ou pâtes dentifrice, de bains de bouche, de sprays, de mousses, de gargarismes, de gels dentaires ou de gommes à mâcher.

7. Composition selon l'une des revendications 1 à 6, caractérisée en ce qu'elle comporte en outre un agent de polissage présent dans des proportions allant jusqu'à 95% en poids par rapport au poids total de la composition.

8. Composition selon l'une des revendications 1 à 7, caractérisée en ce qu'elle comporte en outre d'autres tensio-actifs de nature anionique, non-ionique, zwittérionique, amphotère ou cationique.

9. Composition selon l'une des revendications 1 à 8, caractérisée en ce qu'elle comporte au moins un agent de cohésion, un agent épaississant, un agent édulcorant, humectant ou rafraîchissant, un agent plastifiant, un arôme ou une substance ou un agent aromatisant, peptisant ou sucrant, un agent de sapidité, un agent conservateur, des oligo-éléments, un agent antibactérien différent de la chlorhexidine ou de ses sels, un agent antibiotique, un agent anticaries, une vitamine, un agent antitartre, un agent colorant, un cicatrisant, un vasomoteur, un agent antisaignement, un agent actif sur la gencive, un agent actif inhibiteur de la mauvaise haleine.

## Claims

1. Composition for oral use containing chlorhexidine in a physiologically acceptable vehicle, characterized in that the chlorhexidine and/or at least one of its salts is combined with the combination of:
- an amount a less than 1% by weight of dodecanediol polyglycerolated with 3.5 mol of glycerol, and
- an amount b less than 2% by weight of sodium lauryl sulphate,
the ratio a/b being greater than 0.1 and less than 1 and the sum a + b being less than 2% by weight relative to the total weight of the composition

2. Composition according to Claim 1, characterized in that the composition contains from 0.01 to 0.6 [lacuna] by weight of chlorhexidine and/or of at least one of its salts as active material relative to the total weight of the composition.

3. Composition according to Claim 1 or 2, characterized in that the chlorhexidine salts are chosen from chlorhexidine digluconate, diformate, diacetate, dipropionate, dihydrochloride, dilactate, dinitrate, sulphate and tartrate,

4. Composition according to one of Claims 1 to 3, characterized in that the chlorhexidine salts are chosen from chlorhexidine digluconate, dihydrochloride and diacetate.

5. Composition according to one of Claims 1 to 4, characterized in that the ratio a/b is between 0.2 and 0.5.

6. Composition according to one of Claims 1 to 5, characterized in that it is in the form of toothpastes or toothpaste gels, mouthwashes, sprays, foams, products for gargling, dental gels or chewing gums.

7. Composition according to one of Claims 1 to 6, characterized in that it also contains a polishing agent which is present in proportions ranging up to 95% by weight relative to the total weight of the composition.

8. Composition according to one of Claims 1 to 7, characterized in that it also contains other surfactants of anionic, nonionic, zwitterionic, amphoteric or cationic nature.

9. Composition according to one of Claims 1 to 8, characterized in that it contains at least one cohesion agent, a thickener, a sweetener, a wetting agent or refreshing agent, a plasticizer, an aroma or a flavouring substance or agent, a peptizing agent or sweetener, a flavour enhancer, a preserving agent, trace elements, an antibacterial agent other than chlorhexidine or its salts, an antibiotic agent, an anti-caries agent, a vitamin, an antitartar agent, a dye, a cicatrizing agent, a vasomotor agent, an anti-bleeding agent, an agent which is active on the gums and an active agent for inhibiting bad breath.

## Patentansprüche

1. Zur Verabreichung im Mund vorgesehene Zusammensetzung, umfassend Chlorhexidin in einem physiologisch geeigneten Trägermedium,
dadurch **gekennzeichnet**, daß
das Chlorhexidin und/oder mindestens einer seiner Salze zusammengebracht sind mit der Kombination aus:
- einer Menge a von weniger als 1 Gew.% Dodecandiol, das mit 3,5 Mol Glycerin polyglyceriert ist, und
- einer Menge b von weniger als 2 Gew.% Natriumlaurylsulfat,
wobei das Verhältnis a/b mehr als 0,1 und weniger als 1 und die Summe a + b weniger als 2 Gew.% betragen, bezogen auf das Gesamtgewicht der Zusammensetzung.

2. Zusammensetzung gemäß Anspruch 1,
dadurch **gekennzeichnet**, daß
die Zusammensetzung 0,01 bis 0,6 Gew.% Chlorhexidin und/oder mindestens einer seiner Salze an Aktivmasse enthält, bezogen auf das Gesamtgewicht der Zusammensetzung.

3. Zusammensetzung gemäß Anspruch 1 oder 2,
dadurch **gekennzeichnet**, daß
die Salze von Chlorhexidin aus dem Digluconat, Diformiat, Diacetat, Dipropionat, Dihydrochlorid, Dilactat, Dinitrat, Sulfat und dem Tartrat von Chlorhexidin ausgewählt sind.

4. Zusammensetzung gemäß einem der Ansprüche 1 bis 3,
dadurch **gekennzeichnet**, daß
die Salze von Chlorhexidin aus dem Digluconat, Dihydrochlorid und dem Diacetat von Chlorhexidin ausgewählt sind.

5. Zusammensetzung gemäß einem der Ansprüche 1 bis 4,
dadurch **gekennzeichnet**, daß
das Verhältnis a/b 0,2 bis 0,5 beträgt.

6. Zusammenseztung gemäß einem der Ansprüche 1 bis 5,
dadurch **gekennzeichnet**, daß
sie in Form von Zahnpastengelen oder Zahnpasten, Mundbädern, Spray-Produkten, Schaumprodukten, Produkten zum Gurgeln, Zahngelprodukten oder von Kaugummiprodukten vorliegt.

7. Zusammensetzung gemäß einem der Ansprüche 1 bis 6,
dadurch **gekennzeichnet**, daß
sie ausserdem ein Poliermittel enthält, das in Mengenanteilen bis zu 95 Gew.% vorhanden ist, bezogen auf das Gesamtgewicht der Zusammensetzung.

8. Zusammensetzung gemäß einem der Ansprüche 1 bis 7,
dadurch **gekennzeichnet**, daß
sie ausserdem weitere oberflächenaktive Mittel anionischer, nicht-ionischer, zwitterionischer, amphoterer oder kationischer Art enthält.

9. Zusammensetzung gemäß einem der Ansprüche 1 bis 8,
dadurch **gekennzeichnet**, daß
sie mindestens ein Kohäsionsmittel, Verdickungsmittel, Süßungs-, Feuchtigkeits- oder Erfrischungsmittel, Plastifiziermittel, einen Aromatstoff oder eine aromatisierende Substanz oder ein aromatisierendes Mittel, ein Peptisier- oder Zuckerungsmittel, ein Geschmacksmittel, ein Konservierungsmittel, Oligo-Elemente, ein antibakterielles Mittel, das sich von Chlorhexidin oder seinen Salzen unterscheidet, ein antibiotisches Mittel, ein Antikariesmittel, ein Vitamin, ein Antizahnsteinmittel, ein Farbstoffmittel, ein Vernarbungsmittel, ein Mittel zur Gefäßmotorik, ein blutstillendes Mittel, ein gegenüber dem Zahnfleisch wirksames Mittel und ein Mittel enthält, das als Inhibitor von schlechtem Atem und Mundgeruch wirksam ist.
